# EUROPEAN PATENT SPECIFICATION

(11) **EP 2 559 997 B1**
(45) Date of publication and mention of the grant of the patent: **18.04.2018**
(21) Application number: 12180914.9
(22) Date of filing: 17.08.2012
(51) Int. Cl.: G01N 33/18

(54) **Method and apparatus for capturing and retesting of an online toc excursion sample**
Verfahren und Vorrichtung zum Erfassen und erneuten Testen einer Online-TOC-Exkursionsprobe
Procédé et appareil pour capturer et retester un échantillon d'excursion TOC en ligne

(30) Priority: 19.08.2011 US 201161525530 P
(43) Date of publication of application: 20.02.2013
(73) Proprietor: Hach Corporation, Loveland, CO 80539 (US)
(72) Inventor: Collier, Matthew Grant, Loveland, CO 80538 (US); Garvin, Robert Lee, Loveland, CO 80538 (US); Stange, Terry Gane, Loveland, CO 80538 (US)
(74) Representative: Sandri, Sandro

(56) References cited:
- EP-A1- 2 354 788
- US-A- 5 299 141
- US-A1- 2003 211 626
- US-A1- 2010 279 417
- Hach: "ANATEL PAT700 On-line Total Organic Carbon Analyzer Edition 4" In: "ANATEL PAT700 On-line Total Organic Carbon Analyzer Edition 4", 1 April 2010 (2010-04-01), Hach Company, XP055318062,

## Description

### FIELD OF THE INVENTION

This invention relates to a method of validating a measurement of total organic carbon in a sample of water, to an analyzer for carrying out that method, and also to a computer program product for carrying out the above method.

### BACKGROUND

Total organic carbon (TOC) is the amount of carbon bound in an organic compound. TOC, which is typically measured from part per trillion (ppt) to parts per million (ppm) of carbon, is often used as a non-specific indicator of water quality or cleanliness. That is, for higher numbers of TOC, the higher number of potential organic contaminants exist within the water, and the lower the TOC, the lower number of potential organic contaminants exist within the water.

All TOC analyzers have in common the purpose of oxidizing or decomposing organic contaminants within a water sample to create carbon dioxide (CO₂) and subsequent measurement of CO₂ using conductivity or NDIR detection methods. In the case of low TOC levels (ppt to low ppm), a conventional approach for determining the amount of TOC in a sample of water may include oxidizing the organic carbon to CO₂ using Ultraviolet (UV) light and measuring the conductivity of the water before and after this oxidizing step. The change in conductivity is then converted to a TOC value using various algorithms based on known conductance and temperature data for the conductive products.

There are many applications for instruments capable of measuring the amount of TOC in water. For example, in the pharmaceutical industry, it is important to have ultra low levels of TOC. Therefore, manufacturers regularly monitor the levels of TOC in the water used to produce the pharmaceuticals and/or clean the production equipment.

Ultra-purified water (UPW) systems are typically designed to continuously monitor conductivity (uS/cm) and TOC (ppbC) levels of the water produced by these systems. An example of a continuous online TOC analyzer is the ANATEL PAT700 TOC analyzer sold by Hach Company of Loveland, CO. ANATEL is a registered trademark of Hach Company in the United States and other countries.

The existing ANATEL PAT700 TOC analyzer equipped with Onboard Automated Standards Introduction System (OASIS) allows calibration of the analyzer by drawing small aliquots from standards bottles inserted into the instrument. Grab samples from other parts of the water system also may be collected and manually inserted for testing using this analyzer. Standards or grab samples are drawn in through a needle to an internal oxidation cell where they are exposed to UV light and decomposed to carbon dioxide. A vent needle allows the water sample to be drawn into the analyzer (out of the bottle) without creating a vacuum inside the bottle.

In addition to acting as a sample inlet, OASIS can also act as a sample outlet, by drawing water from an online source and injecting into empty bottles installed in the analyzer, thus allowing for collection of a water sample from the process for testing at another (laboratory) location for confirmation. In this case, the existing OASIS analyzer can be configured to use an excursion capture and validation feature, enabling the analyzer to capture a water sample from the UPW system when a user-defined high TOC or conductivity alarm occurs during online monitoring. With this feature selected, the instrument will fill a plastic or glass bottle with excursion sample water essentially immediately following the alarm. The filling process uses water system line pressure to back flush and fill the bottle which is inserted into the system in an inverted (or substantially inverted) manner. The bottle, which contains a septum in the cap, is inserted into a bottle bay in an inverted fashion such that needles (water inlet/outlet (transmitting) needle and vent needle) pierce the septum.

In the event that the analyzer detects an excursion and/or potential condition that indicates that the conductivity, TOC or other parameter of water is outside an acceptable range, the analyzer activates alarms to notify the water or production facility. The analyzer also automatically collects a sample of water from the system.

The water sample can then be measured off-line. An off-line measurement typically involves transporting the sample of water collected from the ultra purified water systems of interest to a laboratory for analysis.

At least two potential issues arise when conducting such off-line testing. Firstly, the time at which the water sample is tested most likely will not align with occurrence of the real-time excursion originating within the ultra purified water system. For example, minutes, hours or even days could pass after the excursion occurred and the water sample was collected and before it is tested in the laboratory. At that point, the excursion may have disappeared and the water conditions may have returned to normal . This can result in questions about the accuracy and reliability of the monitoring system and/or lead to unresolved concerns regarding the water quality.

Secondly, collection of the sample may introduce additional contaminants to the water-either from contaminants residing in the collection vessel (typically a glass or plastic bottle) or from air-borne organic materials to which the water sample may be exposed during collection. For example, laboratory personnel may accidentally contact the water sample during collection and cause contamination. Such additional TOC contamination can be a few ppbC or it can be several hundred ppbC. In these situations, it is impossible to differentiate the actual TOC from the water system from the TOC contributed by subsequent sample contamination. If the measured TOC is too high (e.g., greater than 500 ppbC), the ultra purified water cannot be used for the production of pharmaceuticals and/or cleaning of such equipment.

Another potential issue with existing analyzers includes the inability to completely fill the collection bottle with water. Because the bottle does not completely fill with water, there may be an insufficient volume of water to properly test the sample.

### SUMMARY OF THE INVENTION

Embodiments of the invention provide an online method of validating whether the analyzer correctly determined an excursion. This online validation method provides its users with real-time information as to whether it is necessary to take corrective action for the water system or whether the initially detected excursion was an anomaly or false alarm. The invention is as defined in claims 1-14. For a better understanding of the embodiments, together with other and further features and advantages thereof, reference is made to the following description, taken in conjunction with the accompanying drawings.

### DESCRIPTION OF THE DRAWINGS

FIG. 1 illustrates examples of excursion capture bottles.
FIG. 2 illustrates an example excursion capture bottle.
FIG. 3 illustrates an exploded view of an example excursion capture bottle.
FIG. 4 illustrates an example excursion capture bottle in a bottle bay of a TOC analyzer.
FIG. 5 illustrates example screen captures of an analyzer user interface.
FIG. 6 illustrates an example method of validating an excursion sample.
FIG. 7 illustrates example analyzer circuitry.

### DETAILED DESCRIPTION

Reference throughout this specification to "excursion" means an unexpected change or perturbation from normal or typical TOC and/or conductivity values in a water system. Excursions may be caused by organic breakthroughs in the water system and most often cause a temporary increase (hours to days) in TOC or conductivity levels.

Reference throughout this specification to an "excursion sample" means a water sample collected during the event or occurrence of a water system TOC and/or conductivity excursion.

Reference throughout this specification to "real-time excursion" means the actual time during which a water system excursion event occurs or takes place.

Reference throughout this specification to "on-line" means a TOC analyzer or other water analytical instrument is directly connected to a water system allowing the analyzer to sample water from a side stream, branch, or "T"-fitting for the purpose of real-time water measurement. On-line measurements are often synonymous with process or in-process measurement.

Reference throughout this specification to "off-line" means a TOC analyzer or other analytical instrument that is physically separated from or not co-located with the water system. Off-line measurements are often synonymous with laboratory measurement.

Reference throughout this specification to a "septum" means a partition or membrane or sealing member. For example, in embodiments disclosed in this specification, a septum that seals a portion of a bottle is disclosed and partitions the interior of a bottle from the exterior of the bottle. In addition to the sealing function, a septum can be pierced (by a needle) to allow transfer of fluid between the interior and exterior of the bottle or vice versa.

Reference throughout this specification to "validate" or "validating" means to confirm, certify, substantiate or verify.

In an existing system, for example an ANATEL PAT700 TOC analyzer, the vent needle does not allow the bottles to fill completely. Accordingly, a vent drop tube or vent insert for the bottle facilitates venting such that the bottle may be filled completely or substantially completely. The user may test the captured excursion samples in the same oxidation chamber of the analyzer, eliminating several potential error sources in such analyses. For example, once the sample is captured, the excursion sample may be re-drawn into the TOC analyzer and analyzed again for validation, using the same analysis or measurement chamber. If the TOC or conductivity results still exceed user-defined levels, the excursion sample has been validated and the user can feel confident the TOC analyzer has accurately measured the sample and the high results are not simply an instrument malfunction or the result of external contamination. Automatic collection (serial or parallel) of multiple excursion samples may be foreseen. For example, in addition to validating the excursion results via reintroduction and testing of the sample in the analyzer from an excursion bottle, the analyzer may capture other excursion sample(s) in other bottles and store the additional sample(s) for additional testing, such as off-line testing (in a laboratory), as needed. No additional external contamination is introduced into the first excursion sample reintroduced into the analyzer because the sample/instrument interface is never broken, eliminating the risk of exposing the sample to airborne organic contaminants. According to the invention, an analyzer for carrying out the method as claimed is foreseen. The excursion bottles may be filled completely or substantially completely via inclusion of an additional venting feature. Off-line analysis may be more accurate by substantially eliminating the air space or "headspace" in the bottle. Eliminating the headspace prevents dissolved organic contaminants in the sample from portioning into the gas phase (evaporating into the air space above the liquid). Additionally, automatically capturing a water sample during an excursion event eliminates the need for facility personnel to be available to troubleshoot the water system. With the sample captured, validated, re-validated (if needed or desired), and additional samples captured, users can wait to investigate these excursions when it is convenient rather than treating these situations as emergencies. Such real-time quality testing and response at the point of production builds quality into the process.

The description now turns to the Figures.

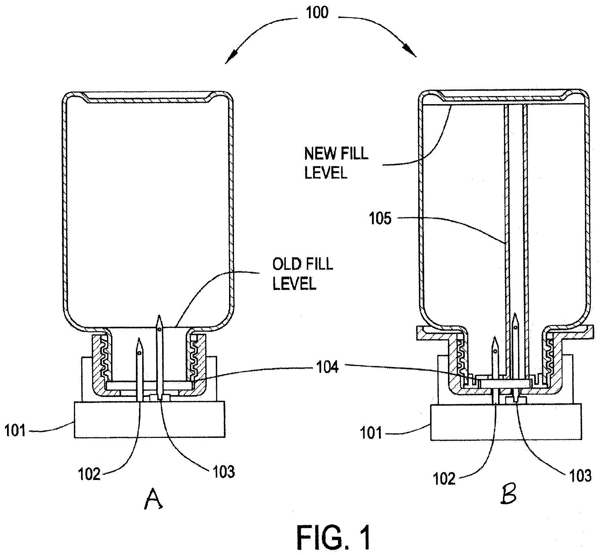

Referring to FIG. 1 (A-B) and 2(A-C), to allow a full bottle 100 of excursion water to be captured, the bottle cap 106 incorporates an internal vent drop tube or insert 105 ("vent tube"), effectively extending the vent needle 103 to the "top" of the bottle 100 (with the understanding that the inverted bottle "top" is the bottom of the bottle when positioned upright). This allows the bottle 100 to fill completely until reaching the level of the effective top of the vent needle 103, with the vent tube 105 providing that effective top, as illustrated in FIG. 1B. By comparison, FIG. 1A illustrates a bottle 100 lacking a vent tube 105. It should be noted throughout that although bottle 100 is referred to as an excursion capture bottle, it may serve other functions, such as a standards bottle, so long as the structure is commensurate with that described herein.

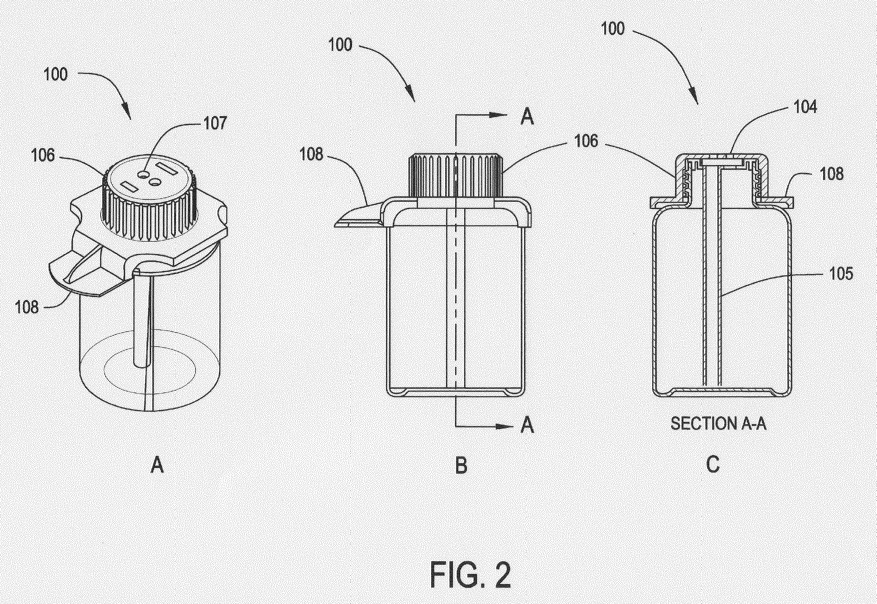

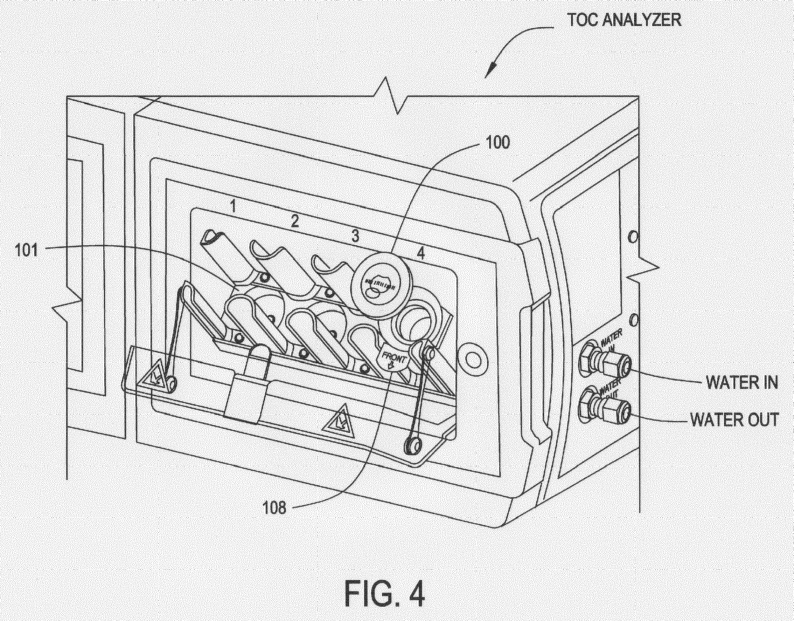

Additionally, the cap 106 structure may include various alignment and orientation tabs 108 to aid in alignment during insertion into the TOC analyzer (FIG. 2 and 4). The cap 106 allows for complete bottle filling at specified water system flow rates, and provides a seal via septum 104.

As illustrated in FIG. 2(B-C), the vent tube 105 extends from the septum 104 to a position near the bottom of the bottle 100 (with the bottle in the upright position). Accordingly, when inverted and inserted into the TOC analyzer (FIG. 4), the inverted bottle is provided with a vent tube 106 that has one end sealing secured to the septum 104 and another end that permits air entry into the vent tube 106 at a position close to the bottle's 100 top (in the inverted position). This permits excursion fluid (for example, water having a TOC content that triggered an excursion sample collection) to fill the bottle to the level of the vent tube 106, that is, a substantially complete filling of the bottle 100.

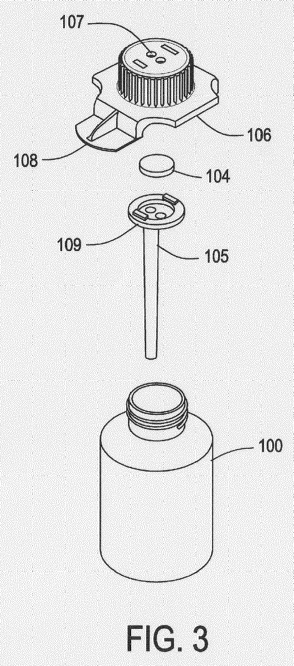

An exploded view is provided in FIG. 3. In the example of FIG. 3, the cap 106 contains needle holes or apertures (vent needle hole is specifically identified at 107) for ease of insertion of the liquid and vent transmitting needles (102, 103 respectively) of the TOC analyzer therein.

The cap 106 may also contain an alignment tab 108 to ensure that the bottle only fits into a corresponding bottle bay (101 of FIG. 1 and FIG. 4) of the TOC analyzer. Thus, the cap 106 may be provided with one or more alignment tabs 108 such that the bottle will only fit into the bottle bay 101 in the proper orientation. This also facilitates proper alignment of needles 102, 103 with their respective holes in the cap 106. Thus, the vent needle 103 will necessarily be aligned with the vent needle hole 107 of the cap 106 by virtue of the alignment tab 108 fitting into correspondingly shaped bottle bay 101 of the TOC analyzer.

Further illustrated in FIG. 3 is septum 104 and vent tube 105. Similar to cap 106 and alignment tab 108 thereof, the vent tube 105 may contain alignment tab(s) 109 to ensure that the vent tube 105 is properly aligned with vent needle hole 107 on assembly. The vent tube 105 may be fitted into place within an interior portion of the cap 106 by aligning the vent tube alignment tab 109 with a corresponding groove in the interior of the cap 106. Thus, the vent tube 106 may be fitted into place within the interior of the cap 106, sandwiching the septum 104 between the vent tube 105 and the cap 106. The septum may comprise a silicone based material with a TEFLON layer positioned on an opposite face of the septum 104 with respect to the cap 106 interior. The septum 104 may be held in position through mechanical means (for example, sandwiched between the vent tube 105 and the cap 106), by means of bonding (chemical or otherwise), or a suitable combination of the foregoing. TEFLON is a registered trademark of E. I. du Pont de Nemours and Company in the United States and other countries.

Once assembled, the bottle 100 is ready to be placed into the TOC analyzer, as illustrated for example in FIG. 4. The example TOC analyzer illustrated in FIG. 4 includes four bottle bays (labeled 1-4), however more or fewer bottle bays may be utilized. The bottle 100 is secured into a bottle bay (4) in the illustration of FIG. 4 in an inverted position with an alignment tab 108 oriented towards the user and having an indicator "front" thereon to inform the user of the proper orientation. Furthermore, an embodiment provides that the door of the TOC analyzer may not be closed if a bottle 100 is secured into the bottle bay 101 (1-4) in an incorrect position. In other words, the TOC analyzer may be configured such that the door of the analyzer will close only when the bottle 100 is oriented in the proper position. Again, the bottle 100 may be a standard bottle, a grab sample bottle, or any like bottle, as described herein.

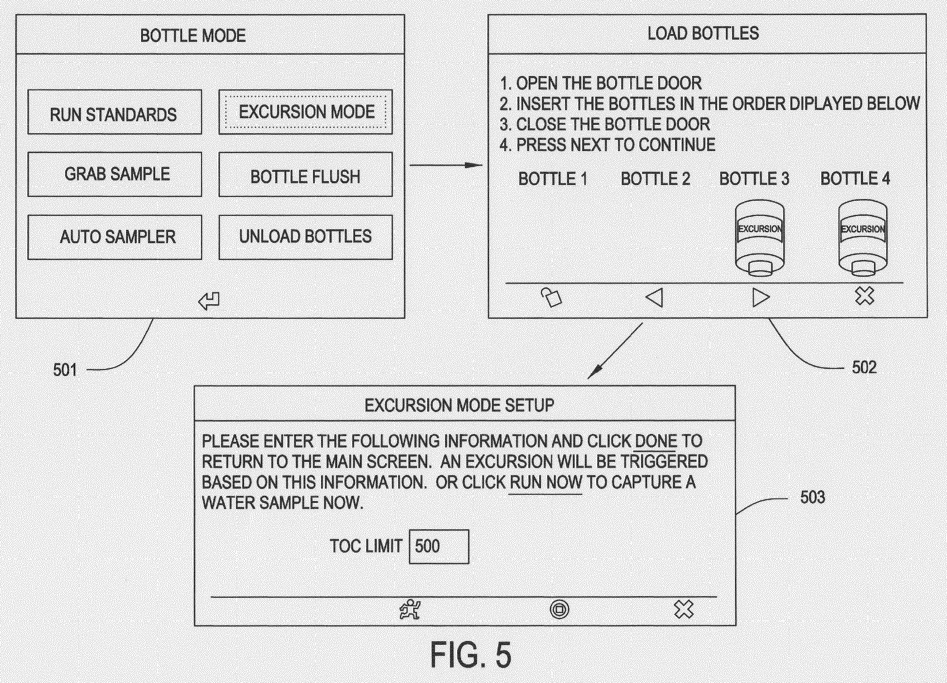

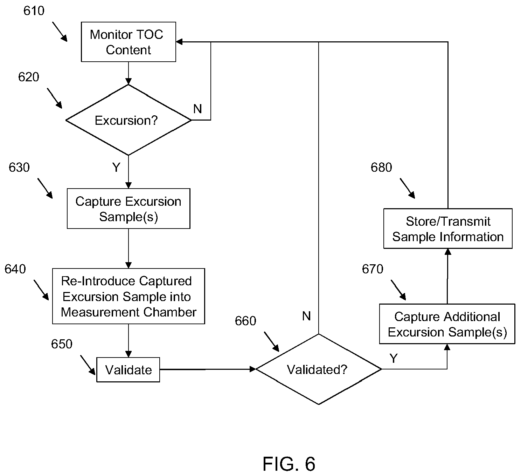

Referring now to FIG. 5 and FIG. 6, re-running a captured excursion sample is possible. FIG. 5 illustrates example screen captures from an example user interface, for example a touch screen user interface. The user begins by configuring the analyzer bottle mode and enabling the analyzer to operate in excursion mode by selecting the excursion mode button on a first interface view 501. Next, the analyzer interface provides a view 502 instructing the user to load the excursions bottles in an appropriate bottle bay (bottle bay locations 3 and 4 in this example). Next, the analyzer interface provides a view 503 instructing the user to enter the TOC trigger limit (in appropriate units) in the excursion mode setup dialog box. The analyzer may also store default value(s) or selectable suggested values for known operations. After pressing the run button, an excursion capture will be triggered based on the trigger limit which was set. Thus, the TOC analyzer has been placed in excursion mode and is prepared to automatically capture an excursion sample in an empty excursion bottle, such as bottle 100, placed into the appropriate bottle bays (bottle bays 3 and 4 in the example of FIG. 4).

In excursion mode, the TOC analyzer may automatically capture one or more excursion samples responsive to a detection that a water sample (flowing through the online analyzer via water in and water out lines, FIG. 4) exceeds the specified TOC limit referred to in connection with FIG. 5. FIG. 6 illustrates an example method of automatically capturing and validating excursion samples.

As the TOC analyzer monitors TOC content of the water flowing through the analyzer 610, either continuously or at predetermined intervals, an excursion may be detected 620. That is, a TOC level exceeding a predefined limit, such as dictated by a user, may be detected 620. If a TOC excursion is detected, an embodiment automatically captures one or more sample bottles of water from the analyzer 630. Thus, the analyzer is capable of capturing a relevant sample of water as an excursion sample in an excursion bottle 100 for further analysis of the excursion event initially detected at 620 from the process water. It should be noted that the TOC analyzer may capture more than one excursion sample responsive to detecting an excursion event, such as serially capturing two or more excursion bottles of sample water (for on board analysis or remote/laboratory analysis). A parallel capture of additional samples may also be utilized. The excursion sample captured in the excursion bottle 100 may be re-introduced into the analyzer to re-analyze or validate the excursion event 640. Thus, a checking or validation mechanism is enabled. This may be performed automatically or may be the result of a manual input by a user, for example as input via a user interface of the TOC analyzer. The sample may be drawn through fluid transmission needle 102, that is, the same needle as used to collect the excursion sample. As described herein, the vent needle 103 provides adequate venting of gas via provision of vent tube 105 to prevent a vacuum in the bottle during excursion sample collection/capture and during reintroduction of the sample.

The TOC analyzer may reanalyze or validate the excursion sample that has been reintroduced to the analyzer using the same analysis chamber, although this is not a requirement 650. If the sample is validated 660, the analyzer may capture additional bottles 670 for further analysis, and store and/or transmit sample analysis information regarding the excursion sample analyses 680. The sample analysis information may be stored in memory, such as that of the TOC analyzer. Moreover, the sample analysis information may he transmitted to another memory device, such as transmitting the sample analysis information to a remote memory device or transmitting the sample analysis information to a storage unit (for example, an RFID tag) attached to the excursion bottle 100 (for example via a sticker containing the RFID tag). The sample information may include but is not limited to TOC online analysis information (the initial detection of TOC excursion), the validation results, bottle identification, process identification, time stamp, location, and the like.

Thus, with excursion mode enabled and two (or more) empty excursion sample bottles loaded, two samples (for example, 65 ml samples) of the process system water may be drawn immediately, filling both bottles, following the trigger event. Once both bottles have been filled, the instrument may automatically run an excursion sample analysis on the contents of one of the bottles (for example, a bottle in bottle bay 3, following the example of FIG. 5). This analysis thus may be used to validate the online (initial) result. Upon analysis of the contents of the excursion capture bottle, the TOC or conductivity results (sample information) may be reported and if the results are the same or substantially the same, a message stating "excursion is valid" will be reported. If the results are not the same, a message stating indicating that the initial reporting of the excursion event was invalid may be provided.

The water sample in the second bottle (for example, bottle in bottle bay number 4 following the example in FIG. 5) is then available for laboratory/offline analysis to help determine the cause of the water system TOC excursion. Excursion sample bottles may contain an RFID tag or other writable storage or printable indicia. The information from the excursion event is written or printed to the bottle's storage device (for example, RFID tag) or printed to attachable indicia (for example, a sticker) to ensure accurate information about the water sample remains available.

The analyzer may then return to online measurement mode and continues to measure TOC and/or conductivity. Further excursion capture is possible if additional bottles are available in unused bottle bays or if the filled excursion bottles are removed and replaced with new, empty excursion capture bottles.

While the example illustrated in FIG. 1 herein illustrates needles 102, 103 that are substantially similar in length, with the vent needle 103 being somewhat longer, it should be noted that the TOC analyzer is not limited to this arrangement. As more or fewer bottle bays may be provided than those illustrated in the example of FIG. 5, so too may needles of differing lengths be provided. For example, needles may be employed such as illustrated in FIG. 2 in certain bottle bays, and needles of differing lengths in other bottle bays. For example, a bottle bay may include a substantially longer vent needle, if desired.

Accordingly, a bottle may be provided for use with an analyzer, comprising: a main bottle body; and a bottle cap attachable to the main bottle body; the bottle cap comprising: a main portion having an aperture therein; a septum sized to cover said aperture and fit within an interior cavity of the bottle cap; and a vent tube disposed within the interior cavity of the bottle cap. The vent tube is disposed within the interior cavity of the bottle cap and extends within the main bottle body when the bottle cap is attached to the main bottle body. Further, the vent tube, the septum and the main portion of the bottle cap may be disposed in a layered structure. The vent tube may be hollow. The bottle cap may further include one or more grooves in an interior therein matching one or more grooves in the vent tube for aligning the vent tube with respect to a vent needle aperture disposed within the bottle cap. The bottle can may further comprise one or more alignment tabs, wherein the one or more alignment tabs are disposed on an exterior surface of the bottle cap. The one or more alignment tabs may be configured to match one or more grooves in a bottle bay of an analyzer. The analyzer may be a total organic carbon analyzer with a storage component configured to store sample information transmitted by the analyzer. The storage component may be an RFID tag.

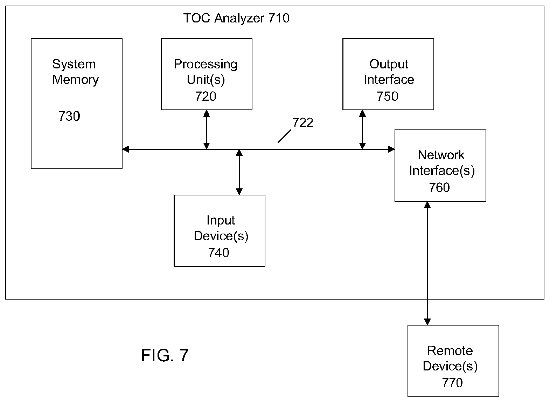

Referring to FIG. 7 and in accordance with the present invention, it will be readily understood that a TOC analyzer ("analyzer") 710 executes program instructions configured to provide automated capturing and testing, as described herein, and perform other functionality of the embodiments.

Components of the analyzer 710 may include, but are not limited to, one or more processing unit(s) 720, a memory 730, and a system bus 722 that couples various components including the memory 730 to the processing unit(s) 720. The analyzer 710 may include or have access to a variety of readable media. The memory 730 may include readable storage media in the form of volatile and/or nonvolatile memory such as read only memory (ROM) and/or random access memory (RAM). By way of example, and not limitation, memory 730 may also include an operating system, application programs, other program modules, and program data.

A user can interface with (for example, enter commands and information) the analyzer 710 through input devices 740. A monitor or other type of device can also be connected to the bus 722 via an interface, such as an output interface 750. In addition to a monitor, analyzers may also include other peripheral output devices. The analyzer 710 may operate in a networked or distributed environment using logical connections to one or more other remote computers or databases. The logical connections may include a network, such local area network (LAN) or a wide area network (WAN), but may also include other networks/buses, including audio channel connections to other devices. Aspects may take the form of an entirely hardware embodiment, an entirely software embodiment (including firmware, resident software, micro-code, et cetera) or an embodiment combining software and hardware aspects. Furthermore, aspects may take the form of a program product embodied in one or more non-signal readable medium(s) having program code embodied therewith.

A combination of readable mediums may be utilized. The readable medium may be a storage medium. A storage medium may be, for example, but not limited to, an electronic, magnetic, optical, electromagnetic, infrared, or semiconductor system, apparatus, or device, or any suitable combination of the foregoing. Examples (a non-exhaustive list) of the storage medium would include the following: a portable memory device, a hard disk, a random access memory (RAM), a read-only memory (ROM), an erasable programmable read-only memory (EPROM or Flash memory), a portable compact disc read-only memory (CD-ROM), an optical storage device, a magnetic storage device, or any suitable combination of the foregoing. In the context of this document, a storage medium may be any non-signal medium that can contain or store a program for use by or in connection with an instruction execution system, apparatus, or device such as analyzer 710.

Program code embodied on a computer readable medium may be transmitted using any appropriate medium, including but not limited to wireless, wireline, optical fiber cable, RF, et cetera, or any suitable combination of the foregoing.

Program instructions may also be stored in a storage medium that can direct an analyzer 710 to function in a particular manner, such that the instructions stored in the storage medium produce an article of manufacture including instructions which implement the function/act specified in this description and/or figures.

## Claims

1. A method of validating a measurement of total organic carbon in a sample of water, comprising:
connecting an analyzer (710) directly to a water flow of an ultrapure water system for monitoring total organic carbon (TOC) in water flowing either continuously or at predetermined intervals through the analyzer;
obtaining a sample of water from the water flow by the analyzer (710);
measuring a TOC amount in a first sample of water using the analyzer (710) and obtaining a first measurement thereof;
when the TOC measured amount in the first sample is above a predefined threshold identifying a potential excursion event;
automatically responding to identification of the potential excursion event by capturing at least one excursion sample of water from the water flow in at least one bottle (100);
introducing the excursion sample from the bottle into the analyzer (710);
measuring a TOC amount in the excursion sample using the analyzer (710) and obtaining a second measurement thereof;
comparing the first measurement and second measurements using the analyzer (710); and
if the first and second measurements are the same or substantially the same reporting that the excursion is valid, or
if the first and second measurements are not the same or substantially the same reporting that the excursion is invalid.

2. The method of claim 1, wherein the capturing step is automated by the analyzer (710).

3. The method of claim 1 or 2, wherein the step of measuring an amount of total organic carbon in the first sample of water occurs when the analyzer is in a first mode.

4. The method of claim 3, wherein the step of measuring an amount of total organic carbon in the excursion sample of water occurs when the analyzer is in the first mode.

5. The method of claim 3, wherein the step of measuring an amount of total organic carbon in the excursion sample of water occurs when the analyzer is in a second mode.

6. The method of any preceding claim, further comprising capturing one or more additional samples of water in corresponding one or more additional bottles (100).

7. The method of claim 6, wherein the one or more additional samples of water are captured in series.

8. The method of claim 6, wherein the one or more additional samples of water are captured in parallel.

9. The method of any preceding claim, further comprising storing sample information comprising one or more of the first measurement, the second measurement and an indication of the comparison.

10. The method of claim 9, wherein the sample information is stored with the bottle (100).

11. The method of claim 10, wherein the sample information is stored with the bottle (100) via transmitting the sample information to an RFID tag of the bottle (100).

12. The method of any preceding claim, wherein the bottle (100) comprises a vent tube (105) disposed within a bottle cap (106) thereof.

13. An analyzer configured to carry
out a method according to any preceding claim, which analyzer comprises one or more processors (720) and a memory (730) storing program instructions executable by the one or more processors (720), the program instructions comprising:
program instructions (610) for measuring an amount of total organic carbon in a first sample of water and obtaining a first measurement thereof;
program instructions (620) for identifying a potential excursion event when an amount of total organic carbon in the first sample is above a predefined threshold;
program instructions (630) for capturing an excursion sample of water in a bottle responsive to detecting the potential excursion event;
program instructions (640) for introducing the excursion sample into the analyser;
program instructions (650) for measuring an amount of total organic carbon in the excursion sample using the analyzer and obtaining a second measurement thereof;
program instructions (660) for comparing the first measurement and second measurement; and
program instructions for if the first and second measurements are the same or substantially the same reporting that the excursion is valid, or
if the first and second measurements are not the same or substantially the same reporting that the excursion is invalid.

14. A program product configured to carry out a method according to any of claims 1-12, the program product comprising:
program instructions (610) for measuring an amount of total organic carbon in a first sample of water and obtaining a first measurement thereof;
program instructions (620) for identifying a potential excursion event when an amount of total organic carbon in the first sample is above a predefined threshold;
program instructions (630) for capturing an excursion sample of water in a bottle responsive to detecting the potential excursion event;
program instructions (640) for introducing the excursion sample into the analyser;
program instructions (650) for measuring an amount of total organic carbon in the excursion sample using the analyzer and obtaining a second measurement thereof;
program instructions (660) for comparing the first measurement and second measurement; and
program instructions for if the first and second measurements are the same or substantially the same reporting that the excursion is valid, or
if the first and second measurements are not the same or substantially the same reporting that the excursion is invalid.

## Patentansprüche

1. Verfahren zur Validierung einer Messung des gesamten organischen Kohlenstoffs in einer Wasserprobe, umfassend:
das direkte Anschließen eines Analysators (710) an einen Wasserstrom eines Reinstwassersystems zur Überwachung des gesamten organischen Kohlenstoffs (TOC) in Wasser, das entweder kontinuierlich oder in vorbestimmten Intervallen durch den Analysator strömt; das Erhalten einer Wasserprobe aus dem Wasserstrom durch den Analysator (710);
das Messen einer TOC-Menge in einer ersten Wasserprobe unter Verwendung des Analysators (710) und Erhalten einer ersten Messung davon;
das Identifizieren eines potenziellen Exkursionsereignisses, wenn die gemessene TOC-Menge in der ersten Probe über einem vordefinierten Schwellenwert liegt;
das automatische Reagieren auf die Identifizierung des potentiellen Exkursionsereignisses durch Erfassen mindestens einer Exkursionsprobe von Wasser aus dem Wasserstrom in mindestens einer Flasche (100);
das Einsetzen der Exkursionsprobe von der Flasche in den Analysator (710);
das Messen einer TOC-Menge in der Exkursionsprobe unter Verwendung des Analysators (710) und Erhalten einer zweiten Messung davon;
das Vergleichen der ersten Messung und der zweiten Messungen unter Verwendung des Analysators (710); und
das Berichten, dass die Exkursion gültig ist, wenn die erste und die zweite Messung identisch sind oder im Wesentlichen identisch sind, oder
das Berichten, dass die Exkursion nicht gültig ist, wenn die erste und die zweite Messung nicht identisch sind oder nicht im Wesentlichen identisch sind.

2. Verfahren nach Anspruch 1, wobei der Erfassungsschritt durch den Analysator (710) automatisiert wird.

3. Verfahren nach Anspruch 1 oder 2, wobei der Schritt des Messens einer Menge des gesamten organischen Kohlenstoffs in der ersten Wasserprobe stattfindet, wenn sich der Analysator in einem ersten Modus befindet.

4. Verfahren nach Anspruch 3, wobei der Schritt des Messens einer Menge des gesamten organischen Kohlenstoffs in der Exkursionsprobe des Wassers stattfindet, wenn sich der Analysator in einem ersten Modus befindet.

5. Verfahren nach Anspruch 3, wobei der Schritt des Messens einer Menge des gesamten organischen Kohlenstoffs in der Exkursionsprobe des Wassers stattfindet, wenn sich der Analysator in einem zweiten Modus befindet.

6. Verfahren nach einem der vorhergehenden Ansprüche, ferner umfassend das Sammeln von einer oder mehreren zusätzlichen Wasserproben in einer oder mehreren zusätzlichen Flaschen (100).

7. Verfahren nach Anspruch 6, wobei die eine oder mehreren zusätzlichen Wasserproben in Serie gesammelt werden.

8. Verfahren nach Anspruch 6, wobei die eine oder mehreren zusätzlichen Wasserproben parallel gesammelt werden.

9. Verfahren nach einem der vorhergehenden Ansprüche, ferner umfassend das Speichern von Probeninformationen, die eine oder mehrere der ersten Messung, der zweiten Messung und einer Angabe des Vergleichs umfassen.

10. Verfahren nach Anspruch 9, wobei die Probeninformationen mit der Flasche (100) gespeichert werden.

11. Verfahren nach Anspruch 10, wobei die Probeninformationen mit der Flasche (100) gespeichert werden, indem die Probeninformation auf ein RFID-Etikett der Flasche (100) übertragen wird.

12. Verfahren nach einem der vorhergehenden Ansprüche, wobei die Flasche (100) ein Entlüftungsrohr (105) umfasst, das in einer Flaschenkappe (106) davon angeordnet ist.

13. Analysator, der konfiguriert ist, um ein Verfahren nach einem der vorhergehenden Ansprüche auszuführen, wobei der Analysator einen oder mehrere Prozessoren (720) und einen Speicher (730) aufweist, der durch den einen oder die mehreren Prozessoren (720) ausführbare Programmanweisungen speichert, wobei die Programmanweisungen Folgendes umfassen:
Programmanweisungen (610) zum Messen einer Menge des gesamten organischen Kohlenstoffs in einer ersten Wasserprobe und Erhalten einer ersten Messung davon;
Programmanweisungen (620) zum Identifizieren eines potentiellen Exkursionsereignisses, wenn eine Menge des gesamten organischen Kohlenstoffs in der ersten Probe oberhalb eines vordefinierten Schwellenwertes liegt;
Programmanweisungen (630) zum Sammeln einer Exkursionsprobe von Wasser in einer Flasche als Reaktion auf das Erkennen des möglichen Exkursionsereignisses;
Programmanweisungen (640) zum Einsetzen der Exkursionsprobe in den Analysator;
Programmanweisungen (650) zum Messen einer Menge des gesamten organischen Kohlenstoffs in der Exkursionsprobe unter Verwendung des Analysators und Erhalten einer zweiten Messung davon;
Programmanweisungen (660) zum Vergleichen der ersten Messung und der zweiten Messung; und
Programmanweisungen für das Berichten, dass die Exkursion gültig ist, wenn die erste und die zweite Messung identisch sind oder im Wesentlichen identisch sind, oder
Berichten, dass die Exkursion nicht gültig ist, wenn die erste und die zweite Messung nicht identisch sind oder nicht im Wesentlichen identisch sind.

14. Programmprodukt, das zum Ausführen eines Verfahrens nach einem der Ansprüche 1 bis 12 konfiguriert ist, wobei das Programmprodukt umfasst:
Programmanweisungen (610) zum Messen einer Menge des gesamten organischen Kohlenstoffs in einer ersten Wasserprobe und Erhalten einer ersten Messung davon;
Programmanweisungen (620) zum Identifizieren eines potentiellen Exkursionsereignisses, wenn eine Menge des gesamten organischen Kohlenstoffs in der ersten Probe oberhalb eines vordefinierten Schwellenwertes liegt;
Programmanweisungen (630) zum Sammeln einer Exkursionsprobe von Wasser in einer Flasche als Reaktion auf das Erkennen des möglichen Exkursionsereignisses;
Programmanweisungen (640) zum Einsetzen der Exkursionsprobe in den Analysator;
Programmanweisungen (650) zum Messen einer Menge des gesamten organischen Kohlenstoffs in der Exkursionsprobe unter Verwendung des Analysators und Erhalten einer zweiten Messung davon;
Programmanweisungen (660) zum Vergleichen der ersten Messung und der zweiten Messung; und
Programmanweisungen für das Berichten, dass die Exkursion gültig ist, wenn die erste und die zweite Messung identisch sind oder im Wesentlichen identisch sind, oder
Berichten, dass die Exkursion nicht gültig ist, wenn die erste und die zweite Messung nicht identisch sind oder nicht im Wesentlichen identisch sind.

## Revendications

1. Procédé de validation d'une mesure du carbone organique total dans un échantillon d'eau, comprenant :
une connexion directe d'un analyseur (710) à un écoulement d'eau d'un système de production d'eau ultra pure pour surveiller le carbone organique total (TOC) dans l'eau s'écoulant soit de façon continue, soit à intervalles prédéterminés à travers l'analyseur ;
une obtention d'un échantillon d'eau en provenance de l'écoulement d'eau par l'analyseur (710) ;
une mesure d'une quantité de TOC dans un premier échantillon d'eau au moyen de l'analyseur (710) et une obtention d'une première mesure de celle-ci ;
quand la quantité de TOC mesurée dans le premier échantillon se trouve au-dessus d'un seuil prédéfini identifiant un événement d'excursion potentiel ;
une réponse automatique à l'identification de l'événement d'excursion potentiel par un prélèvement d'au moins un échantillon d'excursion d'eau en provenance de l'écoulement d'eau dans au moins une bouteille (100) ;
une introduction de l'échantillon d'excursion en provenance de la bouteille dans l'analyseur (710) ;
une mesure d'une quantité de TOC dans l'échantillon d'excursion au moyen de l'analyseur (710) et une obtention d'une deuxième mesure de celle-ci ;
une comparaison de la première mesure et de la deuxième mesure au moyen de l'analyseur (710) ; et
si les première et deuxième mesures sont identiques ou sensiblement identiques, une indication que l'excursion est valide, ou
si les première et deuxième mesures ne sont pas identiques ou sensiblement identiques, une indication que l'excursion est invalide.

2. Procédé selon la revendication 1, dans lequel l'étape de prélèvement est automatisée par l'analyseur (710).

3. Procédé selon la revendication 1 ou 2, dans lequel l'étape de mesure d'une quantité de carbone organique total dans le premier échantillon d'eau a lieu quand l'analyseur se trouve dans un premier mode.

4. Procédé selon la revendication 3, dans lequel l'étape de mesure d'une quantité de carbone organique total dans l'échantillon d'excursion d'eau a lieu quand l'analyseur se trouve dans le premier mode.

5. Procédé selon la revendication 3, dans lequel l'étape de mesure d'une quantité de carbone organique total dans l'échantillon d'excursion d'eau a lieu quand l'analyseur se trouve dans un second mode.

6. Procédé selon l'une quelconque des revendications précédentes, comprenant en outre un prélèvement d'un ou plusieurs échantillons supplémentaires d'eau dans une ou plusieurs bouteilles supplémentaires (100) correspondantes.

7. Procédé selon la revendication 6, dans lequel l'un ou plusieurs échantillons supplémentaires d'eau sont prélevés en série.

8. Procédé selon la revendication 6, dans lequel l'un ou plusieurs échantillons supplémentaires d'eau sont prélevés en parallèle.

9. Procédé selon l'une quelconque des revendications précédentes, comprenant en outre un stockage d'informations d'échantillon comprenant un ou plusieurs de la première mesure, de la deuxième mesure et d'une indication de la comparaison.

10. Procédé selon la revendication 9, dans lequel les informations d'échantillon sont stockées avec la bouteille (100).

11. Procédé selon la revendication 10, dans lequel les informations d'échantillon sont stockées avec la bouteille (100) par transmission des informations d'échantillon à une étiquette RFID de la bouteille (100).

12. Procédé selon l'une quelconque des revendications précédentes, dans lequel la bouteille (100) comprend un tube d'évent (105) disposé dans une capsule de bouteille (106) de celle-ci.

13. Analyseur configuré pour mettre en oeuvre un procédé selon l'une quelconque des revendications précédentes, ledit analyseur comprenant un ou plusieurs processeurs (720) et une mémoire (730) stockant des instructions de programme exécutables par le ou les processeurs (720), les instructions de programme comprenant :
des instructions de programme (610) pour mesurer une quantité de carbone organique total dans un premier échantillon d'eau et obtenir une première mesure de celle-ci ;
des instructions de programme (620) pour identifier un événement d'excursion potentiel quand une quantité de carbone organique total dans le premier échantillon se trouve au-dessus d'un seuil prédéfini ;
des instructions de programme (630) pour prélever un échantillon d'excursion d'eau dans une bouteille en réponse à une détection de l'événement d'excursion potentiel ;
des instructions de programme (640) pour introduire l'échantillon d'excursion dans l'analyseur ;
des instructions de programme (650) pour mesurer une quantité de carbone organique total dans l'échantillon d'excursion au moyen de l'analyseur et obtenir une deuxième mesure de celle-ci ;
des instructions de programme (660) pour comparer la première mesure et la deuxième mesure ; et
des instructions de programme pour que, si la première et deuxième mesures sont identiques ou sensiblement identiques, indiquer que l'excursion est valide, ou
si les première et deuxième mesures ne sont pas identiques ou sensiblement identiques, indiquer que l'excursion est invalide.

14. Produit de programme configuré pour mettre en oeuvre un procédé selon l'une quelconque des revendications 1 à 12, le produit de programme comprenant :
des instructions de programme (610) pour mesurer une quantité de carbone organique total dans un premier échantillon d'eau et obtenir une première mesure de celle-ci ;
des instructions de programme (620) pour identifier un événement d'excursion potentiel quand une quantité de carbone organique total dans le premier échantillon se trouve au-dessus d'un seuil prédéfini ;
des instructions de programme (630) pour prélever un échantillon d'excursion d'eau dans une bouteille en réponse à une détection de l'événement d'excursion potentiel ;
des instructions de programme (640) pour introduire l'échantillon d'excursion dans l'analyseur ;
des instructions de programme (650) pour mesurer une quantité de carbone organique total dans l'échantillon d'excursion au moyen de l'analyseur et obtenir une deuxième mesure de celle-ci ;
des instructions de programme (660) pour comparer la première mesure et la deuxième mesure ; et
des instructions de programme pour que, si les première et deuxième mesures sont identiques ou sensiblement identiques, indiquer que l'excursion est valide, ou
si les première et deuxième mesures ne sont pas identiques ou sensiblement identiques, indiquer que l'excursion est invalide.
